# EUROPÄISCHE PATENTSCHRIFT

(11) **EP 1 499 221 B1**
(45) Veröffentlichungstag und Bekanntmachung des Hinweises auf die Patenterteilung: **27.07.2005**
(21) Anmeldenummer: 02792881.1
(22) Anmeldetag: 04.12.2002
(51) Int. Cl.: A47G 9/02

(54) **ZUDECKE**
BLANKET
COUVERTURE

(30) Priorität: 02.05.2002 DE 10219702
(43) Veröffentlichungstag der Anmeldung: 26.01.2005
(73) Patentinhaber: Sanders GmbH, 49076 Osnabrück (DE)
(72) Erfinder: SANDERS, Hans-Christian, 49076 Osnabrück (DE)
(74) Vertreter: HOFFMANN EITLE
(86) Internationale Anmeldenummer: PCT/EP2002/013701
(87) Internationale Veröffentlichungsnummer: WO 2003/092451

(56) Entgegenhaltungen:
- EP-A- 0 673 613
- WO-A-92/18036
- CH-A- 682 977
- DE-A- 19 828 218
- DE-C- 887 562
- DE-U- 20 102 560
- GB-A- 486 763
- GB-A- 859 071
- GB-A- 2 266 661
- GB-A- 2 295 543
- US-A- 2 368 220
- US-A- 2 808 596
- US-A- 3 325 832
- US-A- 3 717 888
- US-A- 4 324 012
- US-A- 4 525 406
- US-A- 4 839 934
- US-A- 5 181 287
- US-A- 5 386 602
- US-A- 5 669 088

## Beschreibung

Zudecken, wie sie insbesondere für Betten, aber auch für sonstige Möbel vorgesehen sind, sollen leicht und im Sinne einer Anschmiegsamkeit flexibel sein, um sich dem Körper ausreichend anzupassen, während die thermischen Eigenschaften im Hinblick auf die Umgebungstemperatur, insbesondere etwa die Schlafraumtemperatur bei Nacht, dadurch zu berücksichtigen ist, daß man eine mehr oder weniger "warme" Zudecke wählt. Dementsprechend bestehen Zudecken in südlichen Ländern regelmäßig nur aus einer Wolldecke über einem einfachen Bettlaken, die bei kühlerer Umgebungstemperatur durch eine zweite Wolldecke oder weitere Wolldecken zu ergänzen ist. In den mittel- und nordeuropäischen Ländern überwiegen Zudecken, bei denen eine Lage leichten, isolierenden Materials aus einer Steppdecke oder Daunendecke besteht, die insgesamt in einen Bettbezug eingeschlossen ist. Auch dabei gibt es die Möglichkeit, zwischen dickeren bzw. besser isolierenden Lagen und dünneren Lagen zu wählen, um der Umgebungstemperatur Rechnung zu tragen. Die nachfolgende Betrachtung trifft auch auf besondere Formen der Zudecke, insbesondere auch auf Schlafsäcke zu, die in den Begriff "Zudecke" einbezogen sein sollen.

Allerdings hat sich gezeigt, daß ein zufriedenstellendes Liegeklima bzw. Schlafklima damit ggf. nur unzureichend einzustellen ist. Beim Schlafen oder Ruhen kann sich je nach der Transpiration des Liegenden, der Dampfdurchlässigkeit der Zudecke und der Luftfeuchtigkeit der Umgebung ein allgemeiner oder bereichsweiser Körperschweiß ergeben, der sowohl bei kühlem wie auch bei warmen Befinden unangenehm ist. Daneben bewirkt eine Durchfeuchtung der Bekleidung auch gesundheitliche Risiken, wenn danach durch Bloßlegen zufällig eine örtliche Abkühlung entsteht.

Der Mensch schwitzt in der Nacht bis zu ca. 500 ml Wasser aus. Dieses Wasser bzw. der Wasserdampf muß das komplette Bettsystem verlassen, um ein direktes Feuchtigkeitsaufkommen in der Betthöhle zu vermeiden. Der Abtransport kann entweder erfolgen nach unten durch das Auflagesystem, wie z. B. Matratze oder durch die Zudecke bzw. seitliche Öffnungen bei nicht vollkommen umschlossenen Körper.

Es ist bekannt, daß der Hauptteil der Feuchtigkeit durch die Zudecke abwandern muß. Hier trifft die Feuchtigkeit jedoch auf verschiedene Widerstände, zunächst die erste Schicht des Bettbezugs, dann die erste Schicht des Inletts, dann das Füllmaterial, dann die zweite Schicht des Inletts, die zweite Schicht des Bettbezuges und erreicht dann erst die Umgebungsluft. Beim Passieren dieser Schichten findet sowohl eine Speicherung von Feuchtigkeit statt, die wieder weiterzuleiten und abzugeben ist, als auch eine Mischung aus diffusivem und leicht konvektivem Transport. Insgesamt handelt es sich hier jedoch um langsam verlaufende feuchtigkeitsausgleichende Prozesse mit quasi jeweils hohen Einzelwiderständen. Nicht zuletzt die Entwicklung der Inletts für Daunen- oder Faser- sowie Natur- oder Tierhaar gefüllte Zudecken in Richtung einer entsprechenden Daunen- oder Faserdichtigkeit und somit Geweben mit hoher Fadendichte und feinen Einzelfäden, die natürlich eine hohe Dichtigkeit aufweisen hinsichtlich des Durchstechens der einzelnen Füllmedien, jedoch auch eindeutig reduzierten Luftdurchlaß aufweisen. Dies führt zur Verringerung des gesamten Luftaustausches, enthaltend die Feuchtigkeit und zu einer Art Stauwirkung. Die Folge ist, das zeigen auch Untersuchungen verschiedener Institute, daß wir innerhalb der Betthöhle quasi tropische Klimata erhalten. Die Lufttemperaturen liegen grob zwischen 30 und 35 °C bei entsprechend hoher Luftfeuchtigkeit.

Da der Mensch jedoch Unwohlsein bei diesen Klimata empfindet, führt es dazu, daß man nachts entsprechend unruhig schläft, sich herumwälzt und aufdeckt. Nach dem Aufdecken tritt jedoch eine Verdunstungskühlung des ausgeschwitzten Schweißwassers auf und somit eine Abkühlung, die dann wieder dazu führt, daß man sich im Schlaf zudeckt.

US 5,181,287 offenbart eine mit Löchern versehene Decke, bei der die einzelnen Löcher durch sich selbsttätig öffnende Verschlussklappen bedeckt sind. Eine Überspannung der Öffnungen mit einem Netzgitter ist in der Druckschrift nicht offenbart.

US 3,325,832 offenbart eine Zudecke mit einer flexiblen Lage leichten isolierenden Materials, wobei die Lage mit Ventilationsöffnungen versehen ist, die jeweils durch separate Netzgitter überspannt sind.

Insgesamt finden somit unruhige Schlafphasen statt, die die Schlafarchitektur stören.

Der Erfindung liegt dementsprechend die Aufgabe zu Grunde, eine Zudecke zu schaffen, die nicht nur in Bezug auf die thermischen Eigenschaften, d.h. der ausreichenden Isolierung und Abführung überschüssiger Wärme, sondern auch in Bezug auf den Feuchtigkeitstransport insbesondere bei zum Schwitzen neigenden Menschen oder in einem schwülen Raumklima geeignet ist Probleme zu vermeiden, wobei die Handhabung, Benutzung, Reinigung und dergleichen im vertrauten Bereich liegen soll und wobei die. Herstellungskosten in einem annehmbaren Bereich liegen müssen.

Gemäß der Erfindung wird diese Aufgabe mit einer Zudecke nach dem Anspruch 1 gelöst. Diese Zudecke schafft Ventilationsmöglichkeiten für den Abtransport von Luft durch die Zudecke hindurch und ermöglicht damit auch einen Feuchtigkeitsausgleich vom Körper zur Umgebung hin sowie die Abfuhr überschüssiger Wärme (Wärmestau).

Wesentlich bei der Zudecke ist, daß sie keine geschlossene Sperrlage für Luftbewegung darstellt und daß sie gleichwohl in thermischer Sicht eine vorgegebene Isolierung aufrecht erhält.

Der Erfindung lag daher die Idee zugrunde, Ventilationsöffnungen zu verwenden, die einerseits direkte Thermik der warmen Luft und somit einen hohen Abtransport an Luftfeuchtigkeit gewährleisten, weiterhin eine erzwungene Konvektion bei körperlichen Bewegungen und somit Raumvolumenveränderungen in der Betthöhle zulassen, aber dennoch die bekannten Eigenschaften der Anschmiegsamkeit und der Behaglichkeit beibehalten. Dies gelingt durchaus mit bekannten in Anspruch 1 genannten Füllmedien, so daß selbst im Sommer sehr kuschelige, wohlige Zudeckenkonstruktionen möglich sind, die dennoch ein ideales Betthöhlenklima schaffen. Dies führt wiederum zu ruhigerem Schlafverhalten und besserer Ausgeglichenheit und Leistungsfähigkeit sowie möglicherweise auch Gesundheit des Schlafenden.

Bei diesem Zudeckentyp und einer gemäß Anspruch 1 angepaßt konstruktiven Auslegung wird der Hauptteil der ausgeschwitzten Körperflüssigkeit über die Öffnungssysteme ausgetragen und somit auch die Zudecke sehr viel weniger mit Transportaufgaben des Wasserdampfs belastet. Dies führt dazu, daß kein, wie der Volksmund sagt, klammes Bettgefühl mehr entsteht und die Bettdecke sehr viel weniger Zeit zum Ablüften benötigt.

Die vergleichsweise in Betracht zu ziehende Schlafpraxis, etwa bei heißem oder schwülem Wetter nur unter einem Laken oder einem Bettbezug zu schlafen, ist demgegenüber weniger günstig, da die Wärmeisolierung weitestgehend preisgegeben ist, wo ein Laken am Körper anliegt, ohne daß damit aber ein entsprechend leichter Übergang der Luft nach außen möglich wäre.

Bei der erfindungsgemäßen Zudecke sind die Ventilationsöffnungen jeweils durch separate Netzgitter überspannt. Insbesondere können die Ventilationsöffnungen durch weite Netzgitter oder grobmaschiges Material überspannt sein, um auch insbesondere bei größeren oder langgestreckten Öffnungen den Zusammenhalt der Zudecke sicherzustellen. Die flexible Lage isolierenden Materials gemäß Anspruch 1 kann durchaus insbesondere unter hygienischen Gesichtspunkten mit einem Laken oder einem Bettbezug kombiniert werden, sofern sich immer noch durch die Ventilationsöffnungen ein wesentlich erleichternder Luftübergang ergibt, zumal derartige Laken und Bettbezüge regelmäßig nicht aus besonders dichtem Gewebe wie etwa Inlettgewebe gefertigt sind.

Die Durchlässigkeit einer solchen Überspannung ist wesentlich, einerseits die gewünschte Ventilation sicherzustellen und andererseits Effekte wie beim bereichsweisen Bloßliegen zu vermeiden.

In dieser Hinsicht ist es erforderlich, dass die Durchlässigkeit des Netzgitters zwischen 200 l/dm^{2.} min bei bei einem Druckgefälle von 200 Pa und 9000 l/dm^{2.} min bei einem Druckgefälle von 13 Pa liegt, was das Gitter einerseits von dichteren Geweben und andererseits von freien Öffnungen absetzt. Das "Netzgitter" kann dabei im Sinne der leichten Abdeckfunktion vielgestaltig ausgeführt sein. Aus der Praxis heraus finden sich Textilien vor allem im Bereich der Gewirke wie etwa Lochfiletgewirke, insbesondere Polyester-Lochfiletgewirke, oder Charmeuseware wie Rautencharmeuse oder Lochcharmeuse. Auch aus dem Bereich der Gewebe findet sich etwa Gaze oder Gittergewebe als geeignetes Netzgitter. Ferner kommen als Netzgitter textile offene Flächengebilde wie Vliesstoffe, insbesondere thermobondierte Spinnvliesstoffe, ggf. mit Ausstanzungen oder Fasern in Bi- oder Multiaxialgelegen wie sie auch als Verstärkungsmaterial in faserverstärkten Kunststoffen Einsatz finden, in Betracht. Vorteilhafte Beispiele von Netzgittem sind in der Draufsicht bzw. Durchsicht insbesondere mit einem Anteil der Öffnungen wie Löcher, Poren, freier Maschenraum und dgl. von 15% bis 30% zu beobachten.

Die Wirkung der Netzgitter im Sinne einer vorgebbaren Permabilität der Ventilationsöffnungen läßt sich auch durch zusätzliche Füllungen aus Fasermaterial oder leichte, offene Schütt- oder Füllkörper oder offenporige flexible Körper, insbesondere Schaumstoffkörper mit weiten Poren, ergänzen, die einseitig zu einem einzelnen Netzgitter oder aber - vorzugsweise zwischen zwei Netzgitterlagen angeordnet sind. Sie sind dann praktisch Teil eines räumlichen Netzgitters.

Die Ventilationsöffnungen haben vorzugsweise eine Weite von 5 bis 300 mm, auch in Abhängigkeit von einer Stärke der Zudecke zwischen 0,3 und 150 mm. Dabei sollten die Ventilationsöffnungen einen auf die Gesamtfläche des Bettes bezogenen Flächenanteil zwischen 3% und 50%, vorzugsweise zwischen 5% und 20% einnehmen.

Einige nicht erfindungsgemässe Zudecken und einige Ausführungsbeispiele der Erfindung sind in der Zeichnung dargestellt und werden auch nachfolgend näher beschrieben. In der Zeichnung zeigend:
- Fig. 1: Ansicht einer Zudecke (nicht erfindungsgemäß) mit rasterartig verteilten Ventilationsöffnungen
- Fig. 2: Schnitt nach Linie II -II in Fig. 1
- Fig. 3: Zudecke (nicht erfindungsgemäß) mit ungleichmäßiger Verteilung von Ventilationsöffnungen
- Fig. 4: Kassettendecke (nicht erfindungsgemäß) mit Ventilationsöffnungen
- Fig. 5: Zudecke mit rechteckigen, mit Netzgittern überspannten Ventilationsöffnungen
- Fig. 6: Querschnitt durch die Zudecke nach Fig. 5
- Fig. 7: Zudecke mit streifenförmigen Ventilationsöffnungen
- Fig. 8: Querschnitt durch die Zudecke nach Fig. 7
- Fig. 9: Querschnitt durch eine gegenüber Fig. 8 modifizierte Zudecke
- Fig. 10: Zudecke mit einer aus einem langgesteckten Streifen isolierenden Materials gebildeten Lage, fixiert durch ein Gitternetzwerk
- Fig. 11: Zudecke mit seitlichen Ventilationsöffnungen
- Fig. 12: Zudecke aus einer Vielzahl von kissenförmigen Lageteilen auf einem Netzgitter
- Fig. 13: Schnitt durch die Zudecke nach Fig. 12 (vergrößert)
- Fig. 14: Querschnitt durch eine gegenüber Fig. 13 modifizierte nicht erfindungsgemässe Zudecke
- Fig. 15: Zudecke (nicht erfindungsgemäß) mit Verschlusskappen für Ventilationsöffnungen
- Fig. 16: Schnitt nach Linie XVI - XVI in Fig 15.

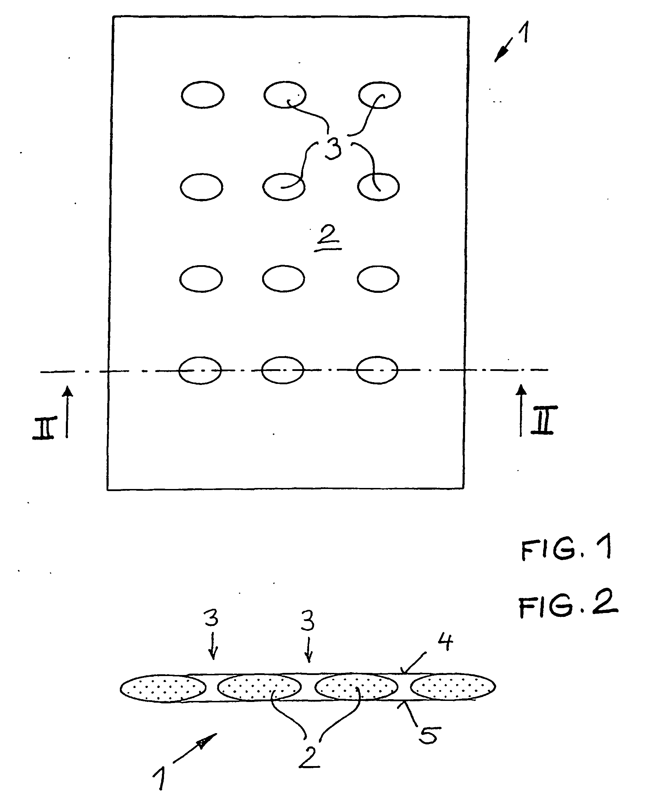

Die in Fig. 1 und 2 insgesamt mit 1 bezeichnete Zudecke (die auf Grund des nicht dargestellten Netzgitters in dieser Form nicht erfindungsgemäß ist) umfaßt eine große zusammenhängende, flexible Lage 2 aus leichtem isolierendem Material, durchbrochen mit rasterartig verteilten ovalen oder runden Löchern 3. Die Lage 2 umfasst eine Inlett-Oberplatte 4 und Unterplatte 5, zwischen denen eine Faser-, Naturhaar-, Tierhaar-, oder Daunenfüllung eingeschlossen ist. Diese Lage aus leichtem isolierendem Material kann auch mit einer reflektierenden Schicht oder Füllung versehen sein, die etwa mit einer feinen Fasermetallisierung für eine Abschirmung gegen Elektrosmog sorgt. Der direkte Einfluß des reflektierenden Anteil auf das Bettklima ergibt sich aber aus der Rückdämmung der Wärmestrahlung. Die Reflektion im infraroten Bereich verbessert und vergleichmäßigt die Isolationswirkung der Lage 2. Die Ventilationsöffnungen 3 übernehmen dabei gleichzeitig die Funktion der Verbindung und Abstandshaltung zwischen Oberplatte 4 und Unterplatte 5, die sonst vielfach durch Stege erzielt wird.

Der wesentliche neue Effekt wird durch die Ventilationsöffnungen 3 erzielt, die einen Luftaustausch zwischen beiden Seiten der Zudecke 1 ermöglichen und damit den Abtransport von Luft mit hoher Körperfeuchtigkeit unter der Zudecke weg ermöglichen. Dieses ist schon durch die Thermik, aber insbesondere dann gegeben, wenn der unter der Zudecke 1 Ruhende Bewegungen durchführt und damit die Räume unter der Decke verändert.

Die Zudecke 1 kann im Bereich der Ventilationsöffnungen 3 im Sinne eines maximalen Luftaustauschs weitgehend freigehalten werden. Grundsätzlich ist aber ein Luftaustausch auch durch übliche textile Hüllmaterialien möglich, wie sie als Spanntücher oder Bettbezüge verwandt werden.

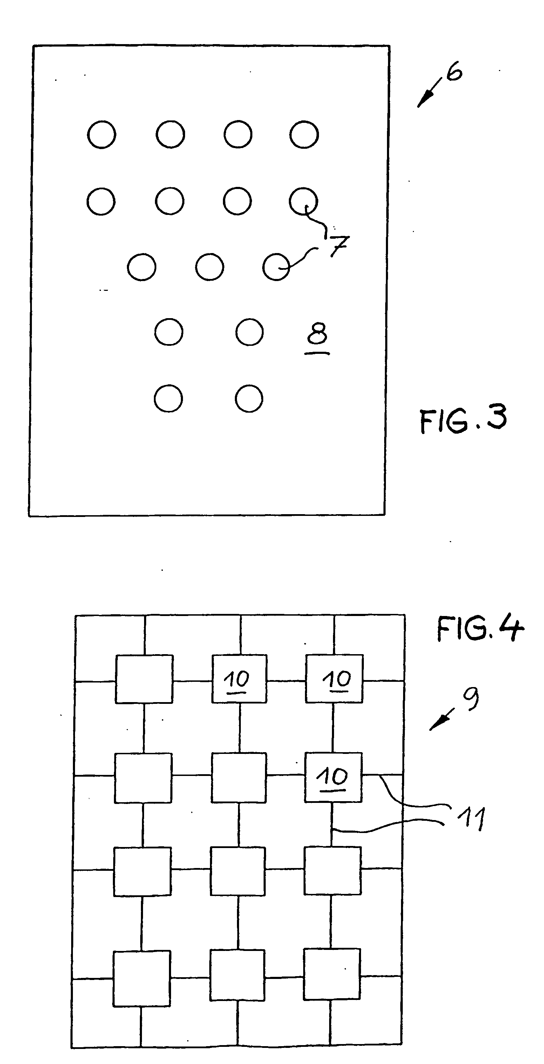

Eine Zudecke 6 nach Fig. 3 (ebenfalls auf Grund der nicht dargestellten Netzgitterüberspannung in dieser Form nicht erfindungsgemäß) weist ungleichförmig verteilte Ventilationsöffnungen 7 auf, die grobhin eine Verteilung der Körpermassen oder -Oberflächen eines Ruhenden widerspiegeln und insbesondere dem bereichsweise unterschiedlichen Bedarf von Luftaustausch angepaßt sind. Diese Ventilationsöffnungen 7 sind wieder in eine Lage 8 leichten isolierenden Materials bzw. herkömmlicher Art einbearbeitet. Eine Zudecke 9 gemäß Fig. 4 (ebenfalls auf Grund der nicht dargestellten Netzgitterüberspannung in dieser Form nicht erfindungsgemäß) ist mit regelmäßig verteilt rechteckigen Ventilationsöffnungen 10 versehen, die in ein Rastersystem einer durch Stege 11 unterteilten Decke, insbesondere Daunendecke eingebunden sind.

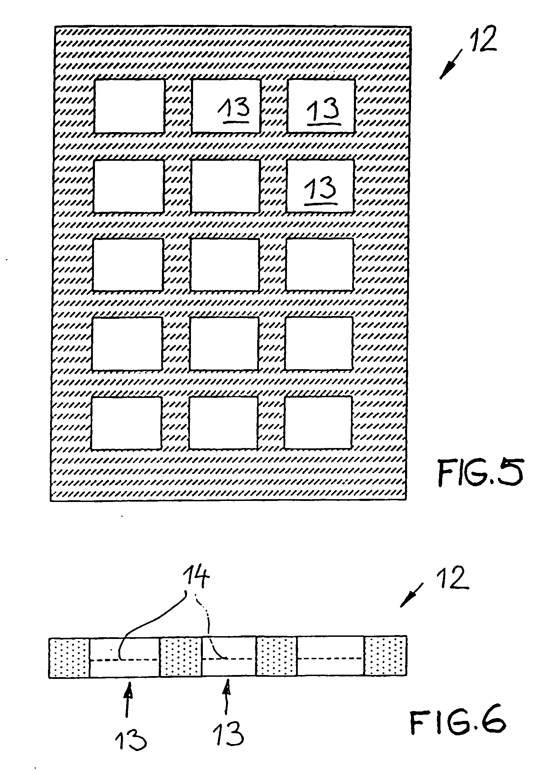

Eine Zudecke 12 gemäß Fig. 5 und 6 mit (beispielsweise) rechteckigen Ventilationsöffnungen 13 ist wiederum von einer beliebigen herkömmlichen Zudecke ausgehend durch die Ventilationsöffnungen 13 geändert zu betrachten, wobei die Ventilationsöffnungen 13 jeweils durch ein Netzgitter 14 überspannt sind, so daß der Zusammenhalt der Zudecke 12 durch die Öffnungen nicht verloren geht und die Zudecke auch optisch einen weiterhin geschlossenen Eindruck vermittelt. Ein solches Netzgitter kann in Form eines Grobgewebes ausgebildet sein, es kann aber auch in einem gelochten Flachmaterial aus Gewebe, Gewirk, Folie oder dergleichen bestehen. Besonders geeignet sind handelsübliche Lochgewirke, deren Löcher und Lochanteil für die gewünschte Permeabilität groß genug vorzugeben sind. Die netzgitterartigen Abdeckungen weisen eine Durchlässigkeit gemäß Anspruch 1 auf und bieten somit für die Ventilation einen passenden Durchgang.

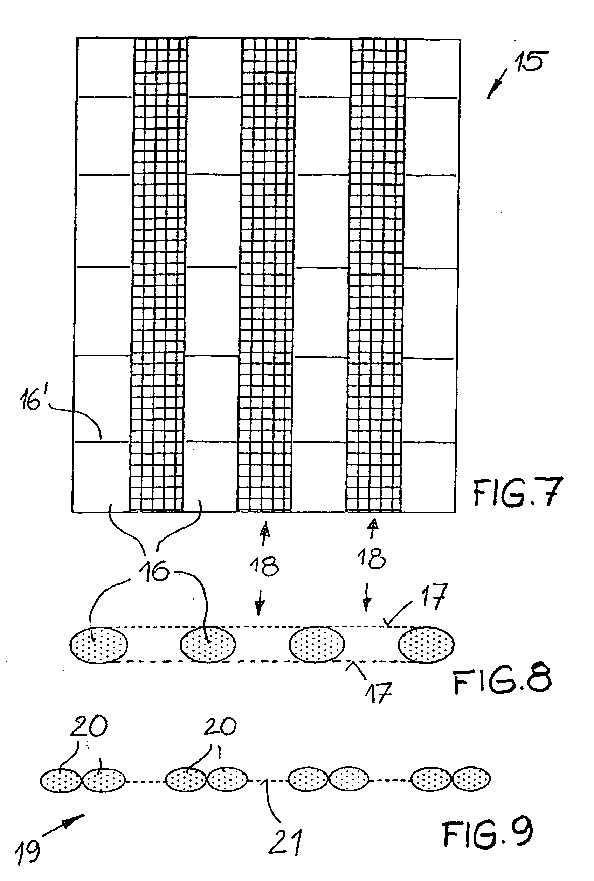

Eine Zudecke 15 nach Fig. 7 und 8 ist aus streifenförmigen Lagen 16 isolierenden Materials mit Querabsteppungen 16' gebildet, die durch durchgehende Netzgitter 17 auf vorgegebene Abstände festgelegt sind, so daß gleichfalls streifenförmige Ventilationsöffnungen 18 entstehen. Dabei versteht es sich, daß normalerweise verhältnismäßig schmale Ventilationsöffnungen bereits eine ausreichende Permeabilität sichern, so daß die dargestellten Proportionen nur ausnahmsweise in Betracht kommen. Eine solche Ausführungsform läßt sich durch die "Elementbauweise" aus den Streifen rationell fertigen.

Die Querschnittszeichnung nach Fig. 9 zeigt eine Zudecke 19, bei der das isolierende Material jeweils in Doppelstreifen 20 vorhanden ist, zwischen denen Streifen 21 eines Netzgitters mit weiten Öffnungen für eine Abstandsfixierung und Ventilation sorgen.

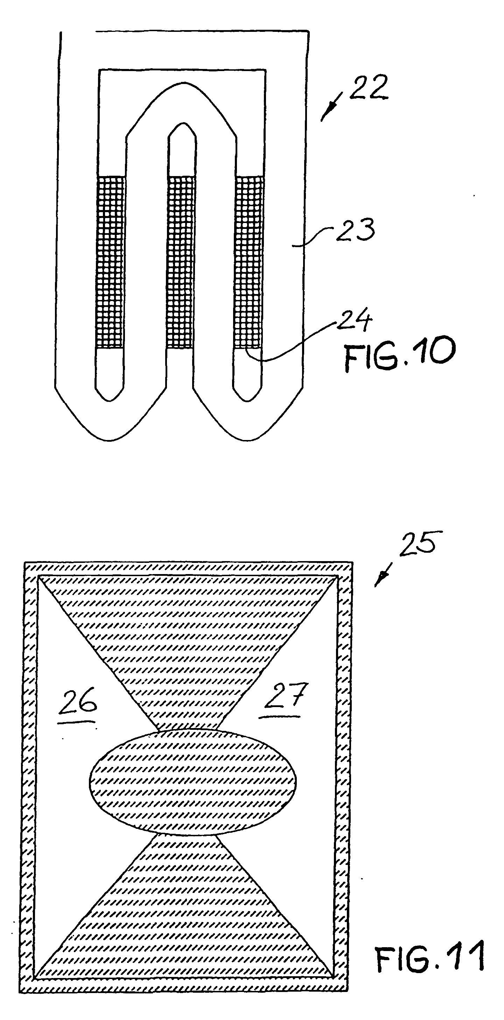

Eine Weiterbildung mit einer Zudecke 22 nach Figur 10 mit einem einzigen, in Schlangenlinien gelegten Streifen 23 isolierenden Materials ist in der "Büroklammerform" durch ein grobmaschiges Netzgitter 24 fixiert, das in einem mittleren Bereich quer über die Windungen verläuft.

In Fig. 11 ist eine Zudecke 25 von einer Rechteckform ausgehend durch große beidseitige Ventilationsöffnungen 26, 27 auf mittlere Bereiche etwa in einer Diabolo-Form reduziert, womit einerseits dem verbleibenden Wärmebedarf des Benutzers andererseits aber auch mit beidseitigen Öffnungen eine gute Ventilation, auch quer durch die Öffnungen 26 und 27 hindurchlaufend, ermöglicht ist. Diese Zudecke 25 wie auch die Zudecke 22 kann z.B. durch einen Bettbezug (nicht dargestellt) in ihrer Permeabilität vorgegeben werden.

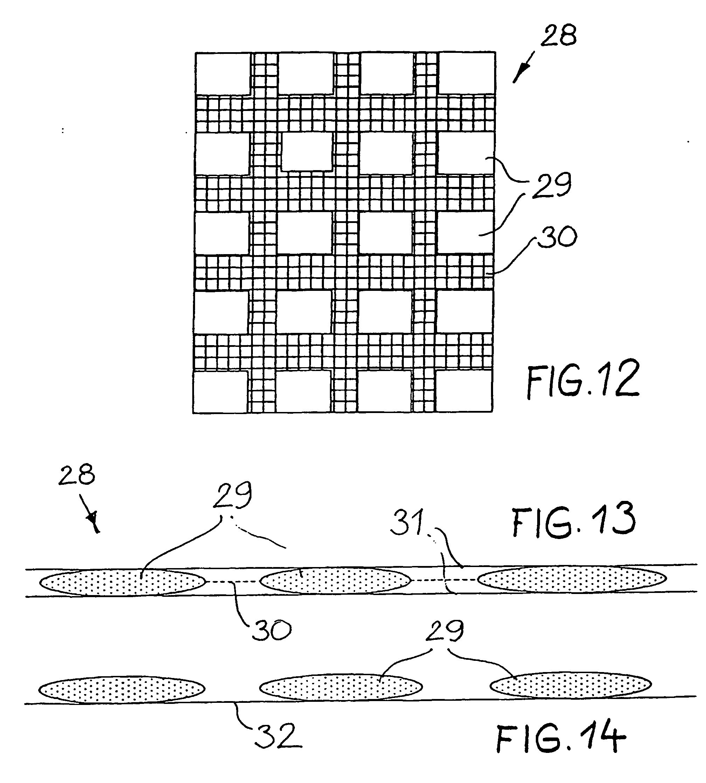

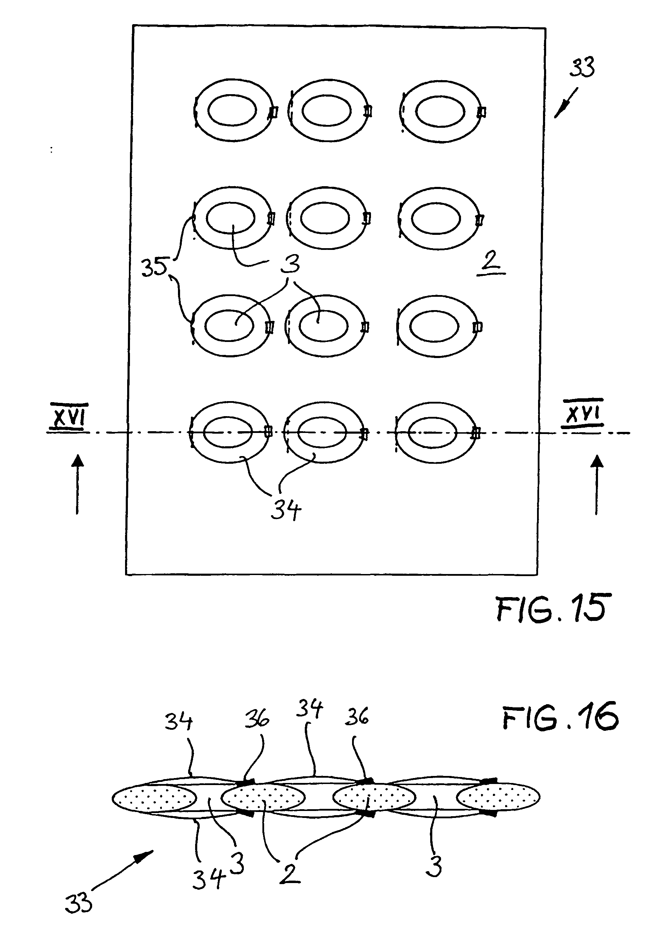

Eine Zudecke 28 gemäß Fig. 12 besteht aus einer in Rechteckelemente 29 zerstückelten Lage isolierenden Materials, die durch ein zwischenliegendes Netz 30 eines Netzgitters miteinander verbunden sind. Die Elemente 29 sind beispielsweise Kissen beliebiger bekannter Füllung. Die gesamte Zudecke 28 kann, wie in der Querschnittszeichnung gemäß Fig. 13 angedeutet, in einen Bettbezug 31 eingezogen sein.

Eine nicht erfindungsgemässe Möglichkeit gemäß Fig. 14 ist die Verbindung der einzelnen Kissen 29 in dem vorgegebenen Abstand mit einem gemeinsamen Netzgitter 32.

In allen vorbeschriebenen Fällen wird eine herkömmlich auf eine gewünschte Isolierung ausgelegte Zudecke mit den Ventilationsöffnungen stärker durchlüftbar, so daß der Abtransport von Feuchtigkeit nicht mehr allein der Saugfähigkeit und der Feuchtigkeitsleitung der Textilen und Füllungen zugedacht wird, sondern eine Ventilation insbesondere bei Körperbewegungen entsteht, mit der feuchtere körpernahe Luft ausgetauscht wird gegen aufnahmefähige Umgebungsluft. Soweit Feuchtigkeit des Körpers auch noch in herkömmlicher Weise von der Lage isolierenden Materials aufgenommen wird, erfolgt die Abgabe an die Umgebungsluft nun nicht nur über die dem Körper abgewandte (Ober-)Seite der Zudecke, sondern auch quer zu den Ventilationsöffnungen hin. Das isolierende Material - etwa Federn oder Fasern - wird trockener gehalten.

In einer in Figur 15 und 16 dargestellten Ausführungsform (ebenfalls auf Grund der nicht dargestellten Netzgitterüberspannung in dieser Form nicht erfindungsgemäß) ist darüber hinaus eine wahlweise Verschließbarkeit der Ventilationsöffnungen vorgesehen. Eine insgesamt mit 33 bezeichnete Zudecke weist eine Lage 2 aus isolierendem Material mit Ventilationsöffnungen 3 auf, die mit denen gemäß Fig. 1 und 2 übereinstimmen können und dementsprechend mit gleichen Bezugszahlen versehen sind. Die Zudecke 33 ist gegenüber der Zudecke 1 nach Fig. 1 und 2 mit Verschlußklappen 34 versehen, die in einem Randbereich jeweils mit einer Naht 35 auf der Lage 2 zu einer Seite an der Ventilationsöffnung 3 festgelegt sind und am gegenüberliegendem Rand zumindest einen Haftverschluß 36 aufweisen, mit dem sie lösbar auf der Lage 2 festzulegen sind und dabei die Ventilationsöffnung 3 abdecken. Der Haftverschluß ist beispielsweise ein Klettverschluß oder Klebeverschluß. Andere Verschlüsse wie Knöpfe oder Druckknöpfe sind grundsätzlich möglich, aber wegen der Ausbildung punktueller harter Oberflächenstellen nicht bevorzugt. Die Verschlußklappen bestehen vorzugsweise aus textilem Material, etwa aus festem Inlettgewebe, und versperren die Ventilationsöffnungen 3, wenn die Zudecke im Winter eher zu kühl erscheint oder wenn ein Schwitzen etwa aus gesundheitlichen Gründen angestrebt wird. Die Verschlußklappen 34 können, wie dargestellt, sowohl auf der Oberseite wie auf der Unterseite der Lage 2 angeordnet sein, so daß die Ventilationsöffnungen nicht nur versperrt, sondern zu Luftkammern umgebildet werden können, die eine noch höhere Isolierwirkung einschalten.

Es versteht sich, daß eine Zudecke mehrere Lagen isolierenden Materials aufweisen kann, um nach Art der herkömmlichen Decken ohne Ventilationsöffnungen bekannten "Duo-Decken" die Isolierwirkung durch die Zahl der jeweils benutzten Lagen und - bei unterschiedlich dicken bzw. unterschiedlich isolierenden Lagen - durch die Auswahl der Lagen vorzugeben. Diese Lagen können dann gemeinsam, etwa randseitig, miteinander vernäht, zusammengeknöpft oder durch Haftverschluß gegeneinander fixiert sein. Sie können auch durch einen Bettbezug zusammengehalten werden.

Diese Variationsmöglichkeiten lassen sich auch bei den erfindungsgemäßen Zudecken anwenden, wobei darüber hinaus die Möglichkeit besteht, zwischen den Lagen die Ventilationsöffnungen zur Deckung zu bringen, um eine möglichst große Ventilation zu erreichen, oder aber mit einem Versatz zwischen den Lagen die Ventilationsöffnungen mehr oder weniger zu schließen oder einen verlängerten Weg durch die Ventilationsöffnungen bei einem Versatz zwischen diesen vorzugeben. Ein solcher Versatz kann durch Haftmittel wie Klettverschlüsse erreicht werden. Es können auch mehrere Knopfreihen oder Knopflochreihen einen wahlweisen Versatz erlauben. Auch bei deckungsgleichen Lagen einer Zudecke kann ein Versatz beispielsweise durch ein Verdrehen einer Lage um 180 Grad erreicht werden, wenn die Ventilationsöffnungen asymmetrisch bzw. nicht punktsymmetrisch auf der jeweiligen Lage verteilt sind.

Bei Steppdecken, bei denen Ober-und Unterplatte im allgemeinen längs der Steppungen direkt (ohne Abstandsstege) miteinander vernäht werden, ergeben sich fertigungsbedingt entsprechend flache Ventilationsöffnungen, insbesondere bei flachem oder volumenarmem Füllmaterial. Um auch hier Ventilationsöffnungen mit einer Durchgangslänge zu erhalten, in der sich die Luft zwar grundsätzlich frei aber beruhigt bzw. verlangsamt bewegt und somit dosiert gehalten wird, haben sich mehrlagige, insbesondere zweilagige Steppdecken mit getrennten Ventilationsöffnungen als vorteilhaft herausgestellt, zwischen denen sich Kammern mit einem darin gehaltenen Luftvolumen bilden.

## Patentansprüche

1. Zudecke (12,15,19,22,25,28), insbesondere für Betten, mit zumindest einer flexiblen Lage (16,23,29) leichten isolierenden Materials, wobei die Lage mit Ventilationsöffnungen (13,18,26,27) versehen ist, die jeweils durch separate Netzgitter (14,17,21,24,30) überspannt sind, **dadurch gekennzeichnet, dass**
(a) die flexible Lage eine Oberplatte und Unterplatte umfasst, zwischen denen eine Faser-, Naturhaar-, Tierhaar- oder Daunenfüllung eingeschlossen ist, und dass
(b) die Durchlässigkeit des Netzgitters zwischen 200 l/dm² · min bei einem Druckgefälle von 200 Pa und 9000 l/dm² · min bei einem Druckgefälle von 13 Pa liegt.

2. Zudecke nach Anspruch 1, **dadurch gekennzeichnet, dass** sie als Schlafsack ausgebildet ist.

3. Zudecke nach einem der Ansprüche 1 bis 2, **dadurch gekennzeichnet, dass** die Ventilationsöffnungen (13) in einem Raster angeordnet sind.

4. Zudecke nach einem der Ansprüche 1 bis 2, **dadurch gekennzeichnet, dass** die Ventilationsöffnungen (18) streifenförmig ausgebildet sind.

5. Zudecke nach einem der Ansprüche 1 bis 2, **dadurch gekennzeichnet, dass** die Ventilationsöffnungen (18) in Form von Zwischenräumen zwischen streifen- oder kissenförmigen Elementen (16,23,29) der isolierenden Lage ausgebildet sind.

6. Zudecke nach einem der Ansprüche 1 bis 5, **dadurch gekennzeichnet, dass** die Ventilationsöffnungen (26,27) ungleichmäßig verteilt sind.

7. Zudecke nach Anspruch 6, **dadurch gekennzeichnet, dass** die Ventilationsöffnungen (26,27) in der Verteilung oder Form auf die menschlichen Körperzonen ausgerichtet sind.

8. Zudecke nach einem der Ansprüche 1 bis 7, **dadurch gekennzeichnet, dass** sie mehrere Lagen aufweist.

9. Zudecke nach Anspruch 8, **dadurch gekennzeichnet, dass** die Ventilationsöffnungen der Lagen untereinander versetzt sind.

10. Zudecke nach einem der Ansprüche 1 bis 9, **dadurch gekennzeichnet, dass** die Lage bzw. zumindest eine Lage elektromagnetische Strahlung reflektierendes Material aufweist.

11. Zudecke nach einem der Ansprüche 1 bis 10, **dadurch gekennzeichnet, dass** den Ventilationsöffnungen zumindest zum Teil Verschlusskappen zugeordnet sind.

12. Zudecke nach Anspruch 11, **dadurch gekennzeichnet, dass** die Verschlusskappen aus textilem Material bestehen und mit Haftverschlüssen festlegbar sind.

## Claims

1. A cover (12, 15, 19, 22, 25, 28), in particular for beds, having at least one flexible layer (16, 23, 29) of light insulating material, wherein the layer is provided with ventilation openings (13, 18, 26, 27) which are respectively spanned by separate meshwork (14, 17, 21, 24, 30),
**characterized in**
a) that the flexible layer comprises an upper plate and a lower plate, between which a filling of fiber, natural hair, animal hair or down is enclosed and
b) that the permeability of the meshwork ranges from 200 l/dm² · min. at a pressure gradient of 200 Pa to 9000 l/dm² · min. at a pressure gradient of 13 Pa.

2. Cover according to claim 1, **characterized in that** it is formed as a sleeping bag.

3. Cover according to one of claims 1 to 2, **characterized in that** the ventilation openings (13) are arranged in a grid pattern.

4. Cover according to one of claims 1 to 2, **characterized in that** the ventilation openings (18) are formed in the shape of strips.

5. Cover according to one of claims 1 to 2, **characterized in that** the ventilation openings (18) are formed in the shape of intermediate spaces between strip-like or cushion-like elements (16, 23, 29) of the insulating layer.

6. Cover according to one of claims 1 to 5, **characterized in that** the ventilation openings (26, 27) are unevenly distributed.

7. Cover according to claim 6, **characterized in that** the distribution or the shape of the ventilation openings (26, 27) is oriented to the zones of the human body.

8. Cover according to one of claims 1 to 7, **characterized in that** it has a plurality of layers.

9. Cover according to claim 8, **characterized in that** the ventilation openings of the layers are offset from each other.

10. Cover according to one of claims 1 to 9, **characterized in that** the layer or at least one layer has a material which reflects electromagnetic radiation.

11. Cover according to one of claims 1 to 10, **characterized in that** closing flaps are allocated at least in part to the ventilation openings.

12. Cover according to claim 11, **characterized in that** the closing flaps consist of textile material and can be fixed with bonding fasteners.

## Revendications

1. Couverture (12, 15, 19, 22, 25, 28), en particulier pour des lits, avec au moins une couche (16, 23, 29) flexible, en matériau léger et isolant, la couche étant munie d'ouvertures de ventilation (13, 18, 26, 27) recouvertes chacune par une grille en filet (14, 17, 21, 24, 30) séparée, **caractérisée en ce que**
(a) la couche flexible comprend une plaque supérieure et une plaque inférieure, entre lesquelles est inclus un remplissage en fibres, en poil naturel, en poil animal ou en duvet, et **en ce que**
(b) la perméabilité de la grille en filet est comprise entre 200 l/dm² . min pour une chute de pression de 200 Pa et 9000 l/dm² . min pour une chute de pression de 13 Pa.

2. Couverture selon la revendication 1, **caractérisée en ce qu'**elle est réalisée sous forme de sac de couchage.

3. Couverture selon l'une des revendications 1 à 2, **caractérisée en ce que** les ouvertures de ventilation (13) sont disposées selon un motif tramé.

4. Couverture selon l'une des revendications 1 à 2, **caractérisée en ce que** les ouvertures de ventilation (18) sont réalisées en forme de bandes.

5. Couverture selon l'une des revendications 1 à 2, **caractérisée en ce que** les ouvertures de ventilation (18) sont réalisées sous forme d'espaces intermédiaires, entre des éléments (16, 23, 29), en forme de bandes ou en forme de coussins, de la couche isolante.

6. Couverture selon l'une des revendications 1 à 5, **caractérisée en ce que** les ouvertures de ventilation (26, 27) sont réparties irrégulièrement.

7. Couverture selon la revendication 6, **caractérisée en ce que** les ouvertures de ventilation (26, 27) sont orientées en répartition ou en forme sur les zones corporelles humaines.

8. Couverture selon l'une des revendications 1 à 7, **caractérisée en ce qu'**elle présente plusieurs couches.

9. Couverture selon la revendication 8, **caractérisée en ce que** les ouvertures de ventilation des couches sont décalées les unes des autres.

10. Couverture selon l'une des revendications 1 à 9, **caractérisée en ce que** la couche, ou au moins une couche, présente un matériau réfléchissant le rayonnement électromagnétique.

11. Couverture selon l'une des revendications 1 à 10, **caractérisée en ce que**, au moins en partie, des bouchons de fermeture sont associés aux ouvertures de ventilation.

12. Couverture selon la revendication 11, **caractérisée en ce que** les bouchons de fermeture sont formés d'un matériau textile et sont susceptibles d'être fixés à l'aide de fermetures adhésives.
